# EUROPÄISCHE PATENTSCHRIFT

(11) **EP 1 108 719 B1**
(45) Veröffentlichungstag und Bekanntmachung des Hinweises auf die Patenterteilung: **27.08.2003**
(21) Anmeldenummer: 00126926.5
(22) Anmeldetag: 08.12.2000
(51) Int. Cl.: C07D 317/12, A61K 7/46, C11B 9/00

(54) **2-Methyl-4-phenyl-1, 3-dioxolan**
2-methyl-4-phenyl-1,3-dioxolane
2-méthyl-4-phényl-1,3-dioxolane

(30) Priorität: 17.12.1999 DE 19961431
(43) Veröffentlichungstag der Anmeldung: 20.06.2001
(73) Patentinhaber: Dragoco Gerberding & Co Aktiengesellschaft, 37603 Holzminden (DE)
(72) Erfinder: Pickenhagen, Wilhelm, Dr., 37671 Höxter (DE); Schatkowski, Dietmar, 37627 Stadtoldendorf (DE)
(74) Vertreter: Eisenführ, Speiser & Partner

(56) Entgegenhaltungen:
- DE-C- 109 176
- MAKOTO HOJO ET AL.: "A NEW AND MILD SYSTEM FOR THE GENERATION OF NONSTABILIZED CARBONYL YLIDES" JOURNAL OF ORGANIC CHEMISTRY., Bd. 62, Nr. 25, 1997, Seiten 8610-11, XP002169753 AMERICAN CHEMICAL SOCIETY. EASTON., US ISSN: 0022-3263
- CHEMICAL ABSTRACTS, vol. 84, no. 13, 1976 Columbus, Ohio, US; abstract no. 90042b, K.KULKA: "ACETALS AND KETALS OF 1-PHENYL-1,2-ETHANEDIOL." Seite 518; Spalte 2; XP002169754 & INT. CONGR. ESSENT. OILS(PAP.), Bd. 6TH, Nr. 72, 1974, Seiten 1-11,

## Beschreibung

Die vorliegende Erfindung betrifft die Verbindung 2-Methyl-4-phenyl-1,3-dioxolan, sowie bestimmte diastereomere Enantiomerenpaare und Enantiomere dieser Verbindung.

2-Methyl-4-phenyl-1,3-dioxolan ist bekannt. Bereits 1900 wurde die deutsche Patentschrift 109176 (Albert Verley) veröffentlicht, in der die Darstellung von 2-Methyl-4-phenyl-1,3-dioxolan in stark protischen Medien, nicht jedoch die Isomerenverteilung des erhaltenen Gemisches beschrieben ist; der Geruch des hinsichtlich seiner Zusammensetzung nicht näher definierten 2-Methyl-4-phenyl-1,3-dioxolan wird in der DE 109176 als Jasmingeruch bezeichnet.

K. Kulka und J.W. Dittrick berichteten im Jahre 1975 ausführlich über die sensorischen Eigenschaften einer ganzen Reihe von synthetisierten Acetalen [K. Kulka, J.W. Dittrick, Cosmetics & Perfumery, Vol. 90, 90-95, (1975)]. Die sensorische Bewertung der synthetisierten Acetale wurde allerdings wiederum nur anhand der Diastereomerengemische vorgenommen. Eine Isolierung der Epimeren oder Enantiomeren aus den Gemischen bzw. deren Synthese unterblieb ebenso wie die sensorische Bewertung der Epimeren oder Enantiomeren.

2-Methyl-4-phenyl-1,3-dioxolan (als Diastereomerengemisch wie in der DE 109176 beschrieben) ist derzeit trotz seiner langen Bekanntheit kein kommerziell verfügbarer Duftstoff. Dies liegt insbesondere an der Anwesenheit unerwünschter Schokoladen-Noten, die mit dem Jasmin-Grundgeruch nicht harmonieren und einen unsauberen sensorischen Gesamtcharakter verursachen.

Es war daher die Aufgabe der vorliegenden Erfindung, einen sensorisch aktiven Stoff (Duft- und/oder Aromastoff) anzugeben, der einen starken blumigen und/oder Jasmin-Geruch besitzt, aber im Vergleich mit dem Geruch von 2-Methyl-4-phenyl-1,3-dioxolan (wie in der DE 109176 oder von Kulka und Dittrick in Cosmetics & Perfumery beschrieben) nur abgeschwächte oder gar keine Schokoladen-Noten aufweist.

Diese Aufgabe wird durch Angabe der bislang weder isolierten noch hinsichtlich ihrer sensorischen Eigenschaften untersuchten Enantiomeren (2R,4S)-2-Methyl-4-phenyl-1,3-dioxolan und (2S,4R)-2-Methyl-4-phenyl-1,3-dioxolan gelöst. Beide Verbindungen besitzen eine starke sensorische Aktivität und überraschenderweise einen Geruch, der keine störende Schokoladen-Note umfaßt. Die erfindungsgemäßen Enantiomere können daher in Parfümkompositionen beliebigen Geruchstyps sowohl als eine der Hauptkomponenten als auch im Spurenbereich vorteilhaft eingesetzt werden. Ebenso ist eine Verwendung in Aromakompositionen möglich.

Die Erfindung beruht auf der überraschenden Erkenntnis, daß Diastereomerengemische des 2-Methyl-4-phenyl-1,3-dioxolan je nach Wahl der Reaktionsbedingungen über ganz eigentümliche geruchliche Eigenschaften verfügen und sich deutlich voneinander unterscheiden.

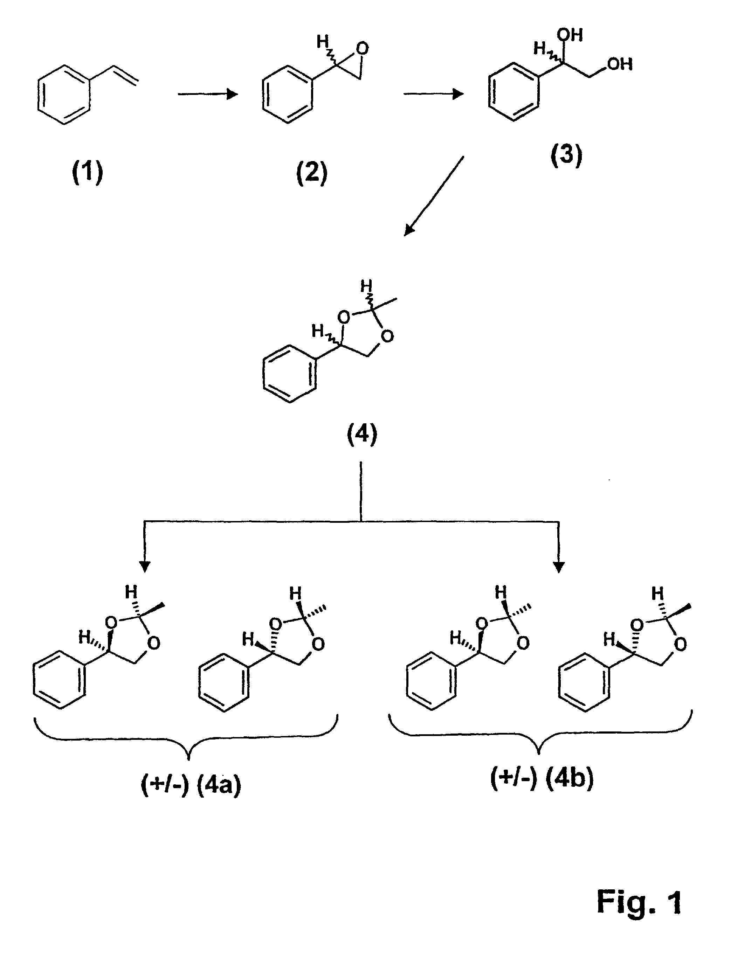

Insbesondere wurde gefunden, daß Diastereomerengemische des 2-Methyl-4-phenyl-1,3-dioxolan (vgl. die allgemeine Formel **(4)** in Fig. 1), die über einen vergleichsweise hohen Anteil an cis-lsomeren (vgl. Formel **(4a)** in Fig. 1) verfügen, sensorisch wertvoller sind als Diastereomerengemische, die über einen besonders hohen Anteil an trans-Isomeren (vgl. Formel **(4b)** in Fig. 1) verfügen.

Bevorzugte sensorisch aktive Stoffe (bevorzugte Duft- oder Aromastoffe) sind Gemische, die einen Gewichtsteil an cis-Enantiomeren **(4a),** d.h.
(2R,4S)-2-Methyl-4-phenyl-1,3-dioxolan und/oder
(2S,4R(-2-Methyl-4-phenyl-1,3-dioxolan
und weniger als zwei, vorzugsweise weniger als einen Gewichtsteil an trans-Enantiomeren **(4b),** d.h.
(2R,4R)-2-Methyl-4-phenyl-1,3-dioxolan und/oder
(2S,4S)-2-Methyl-4-phenyl-1,3-dioxolan umfassen. Das Gewichtsverhältnis von cis-Enantiomeren **(4a)** zu trans-Enantiomeren **(4b)** sollte also zumindest größer als 1:2, vorzugsweise aber sogar größer als 1:1 sein.

Das bevorzugte Gemisch der cis-lsomeren **(4a)** besitzt einen starken, animalischen, narkotisch-grünen, an Hyazinthe erinnernden Geruch. Der Nachgeruch weist dabei deutlich holzige, ozonige Geruchseigenschaften auf.

Hingegen besitzt das weniger bevorzugte Gemisch der trans-Isomeren **(4b)** schwache, leicht animalische Geruchseffekte mit deutlich ausgeprägten Geruchseindrücken, deutlich an Schokolade erinnernd.

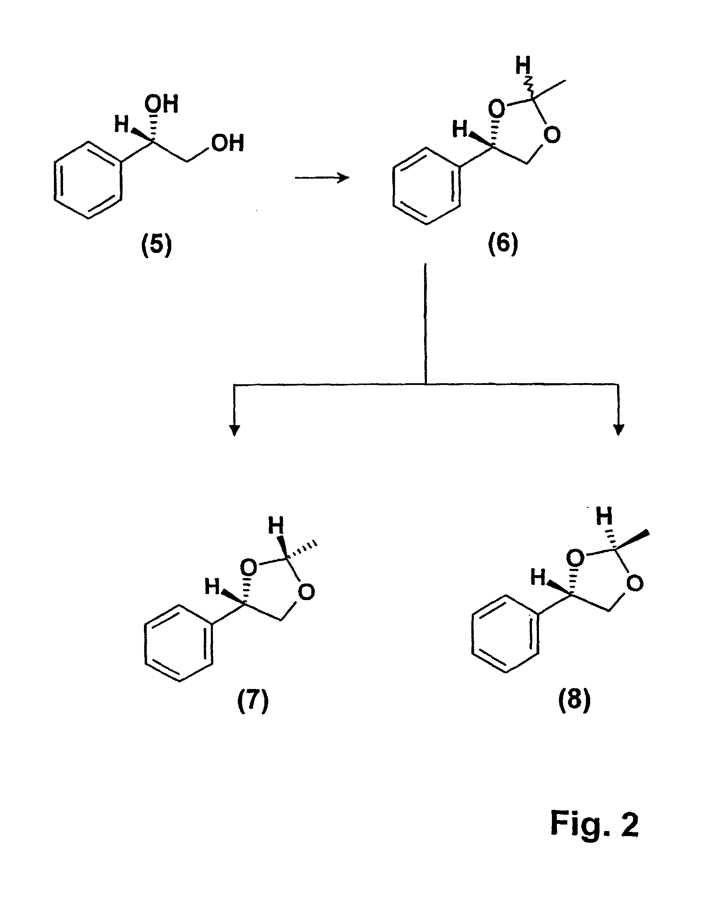

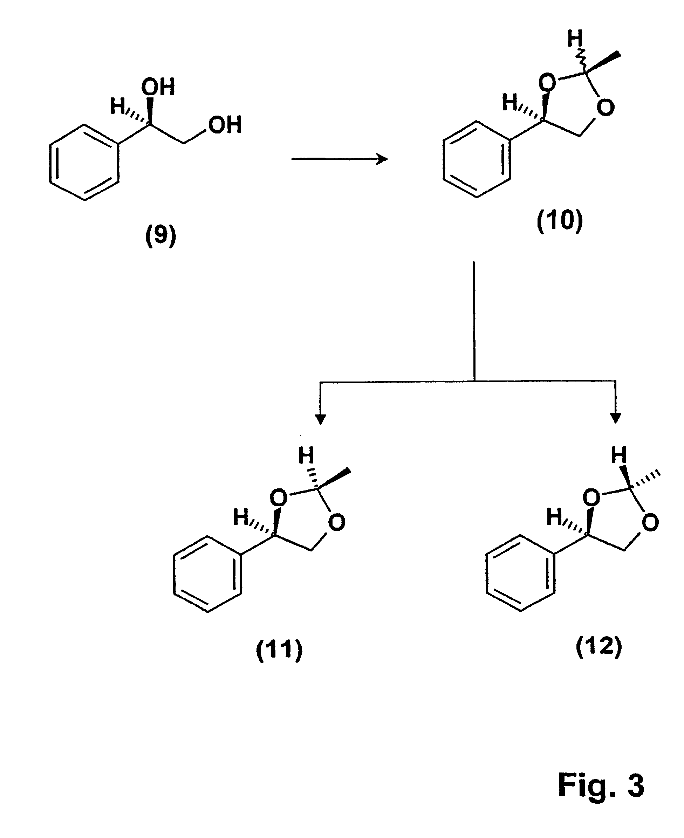

Ebenfalls deutlich unterscheidbar sind die sensorischen Eigenschaften der aus den Epimerenreihen zugänglichen reinen Enantiomeren
(2R,4S)-2-Methyl-4-phenyl-1,3-dioxolan (cis; vgl. Formel **(7)** in Fig. 2),
(2S,4R)-2-Methyl-4-phenyl-1,3-dioxolan (cis; vgl. Formel **(11)** in Fig. 3)
bzw.
(2R,4R)-2-Methyl-4-phenyl-1,3-dioxolan (trans; vgl. Formel **(8)** in Fig. 2),
(2S,4S)-2-Methyl-4-phenyl-1,3-dioxolan (trans; vgl. Formel **(12)** in Fig. 3)

So wurde das gemäß Fig. 2 und den nachfolgendenen Beispielen 3 bzw. 5 aus S(+)-1-Phenyl-1,2-ethandiol zugängliche Acetal **7** (vgl. Fig. 2) mit einer starken, klaren, indoligen Note, die über deutliche Aspekte von Rose und Jasmin verfügt, als das am besten riechende und aufgrund der Geruchsschwelle als das stärkste Isomer beurteilt. Zur Geruchsbewertung der einzelnen Enantiomere vgl. unten die Beispiele 13 und 14.

Zur Herstellung von 2-Methyl-4-phenyl-1,3-dioxolan der allgemeinen Formel **(4)** (Fig. 1) wurde Styrol **(1)** auf übliche Weise in das Styroloxid **(2)** und dieses in ebenfalls bekannter Weise in das 1-Phenyl-1,2-ethandiol **(3)** überführt. Dieses ließ sich dann in bekannter Weise in ein Gemisch **(4)** der diastereomeren Acetale **(+/-) 4a/ (+/-) 4b** (Fig. 1) überführen.

Ausgehend von den kommerziell verfügbaren enantiomerenreinen Diolen **5** (Fig. 2) und **9** (Fig. 3) waren die epimeren Acetale **7, 8** bzw. **11, 12** erhältlich.

Die Isomerenzusammensetzung der synthetisierten Acetale **(+/-) 4a, (+/-) 4b; 7, 8; 11, 12** unterliegt - wie insbesondere ein Vergleich der nachfolgenden Beispiele 2, 9, 10, 11 und 12 zeigt - einem starken Temperatureffekt. Tiefe Temperaturen begünstigen die Bildung der sensorisch wertvolleren cis-Verbindungen (**(+/-) 4a, 7, 11),** hohe Temperaturen hingegen die der sensorisch unerwünschten trans-Verbindungen **(+/-)4b, 8, 12.** So wurde bei - 70 °C ein Diastereomerengemisch mit einem Gewichtsverhältnis von 90:10 (**(+/-) 4a** : **(+/-) 4b** (Beispiel 12)) erhalten, in siedendem Xylol (160 °C) hingegen lediglich ein Diastereomerengemisch mit einem Gewichtsverhältnis von 38:62 (**(+/-) 4a : (+/-) 4b** (Beispiel 10)). Ausgehend von den entantiomerenreinen Diolen wurden entsprechende Isomerengemische **(7:8; 11:12)** gefunden. Dabei besteht in Abhängigkeit von der Temperatur ein fliessender Wechsel zwischen den Isomerenverhältnissen.

Die Acetal-Bildungsreaktionen wurden in Anwesenheit protischer Säuren wie z. B. HCI, H₂SO₄ oder para-Toluolsulfonsäure und in üblichen Lösungsmitteln wie z. B. Hexan, Toluol, Diethylether oder Methanol durchgeführt.

Aus den racemischen bzw. epimerenreinen Diastereomeren-Gemischen wurden die vier einzelnen Enantiomeren durch chromatographische bzw. destillative Methoden in reiner Form gewonnen. Aus den reinen Enantiomeren sind Mischungen mit beliebigen Mischungsverhältnissen herstellbar. Aufgrund der gefundenen Ergebnisse sind (insbesondere zur Herstellung von Duftstoff-Kompositionen) Mischungen mit einem hohen Anteil an den cis-Verbindungen **(+/-) 4a, 7, 11** bevorzugt.

Es sei noch darauf hingewiesen, daß zur Herstellung der sensorisch wertvollen Verbindungen bzw. Verbindungsgemische
(2R,4S)-2-Methyl-4-phenyl-1,3-dioxolan **(7)** und/oder
(2S,4R)-2-Methyl-4-phenyl-1,3-dioxolan **(11)**
auch die jeweiligen Epimeren mit gleicher C4-Konfiguration
(2S,4S)-2-Methyl-4-phenyl-1,3-dioxolan **(8)** und/oder
(2R,4R)-2-Methyl-4-phenyl-1,3-dioxolan **(12)**, gelöst in einem Lösungsmittel, auf eine Temperatur von 20°C oder weniger, vorzugsweise 0°C oder weniger, gebracht und dann so lange bei dieser Temperatur gehalten werden können (bzw. kann), bis sich die gewünschte oder maximale Menge des Zielprodukts gebildet hat.

Die nachfolgenden Beispiele dienen der Illustration der Erfindung:

### Beispiel 1

### Herstellung von (±)-1-Phenyl-1,2-ethandiol (3) - Fig. 1

In einem 2 l Rührwerk mit Rückflusskühler, Thermometer und Tropftrichter wurden 600 g 0,1 %ige H₂SO₄ und 2 g Tetrabutylammoniumhydrogensulfat vorgelegt und anschliessend bei 20 °C bis 25 °C innerhalb von 1 Std. mit 200 g (1,67 mol) Styroloxid **(2)** versetzt. Nach vollendetem Zutropfen wurde 2 Std. nachgerührt und anschliessend aufgearbeitet. Das Reaktionsgemisch wurde mit 200 ml Diethylether versetzt, die organische Phase mit Sodalösung und Wasser neutral gewaschen, über Na₂SO₄ getrocknet und das Lösungsmittel unter reduziertem Druck abdestilliert. Es verblieben 223 g Rohprodukt (91 %ig nach GC).

Gaschromatogramm (Shimadzu GC14A DB1, 30 N, 30 m, 100 - 240 °C, 10 °C x min.⁻¹).
Das Rohprodukt war fest (Smp.: 65 - 67 °).

### Beispiel 2

### Herstellung von (±)-2-Methyl-4-phenyl-1,3-dioxolan (4) (Reaktionstemperatur: 5°C bis 20 °C) - Fig. 1

In einem 1 I-Rührwerk mit Rückflusskühler,Thermometer und Tropftrichter wurden 223 g (1,47 mol) (±)-1-Phenyl-1,2-ethandiol **(3)** (91 %ig nach GC) aus Beispiel 1, 100 ml Diethylether und 2 g para-Toluolsulfonsäure vorgelegt, auf 5 - 10 °C heruntergekühlt und bei dieser Temperatur innerhalb von 30 Min. mit 80,8 g (1,83 mol) Acetaldehyd versetzt. Nach vollendetem Zutropfen rührt man zunächst 4 Std. bei 5 - 10 °C und weitere 2 Std. bei max. 20 °C nach. Nach dieser Zeit wurde mit Wasser versetzt, die organische Phase abgetrennt, die wässrige Phase 1 mal mit 100 ml Ether extrahiert, die vereinigten organischen Phasen mit Sodalösung und Wasser neutralgewaschen. Anschliessend wurde über Na₂SO₄ getrocknet und das Lösungsmittel unter vermindertem Druck abdestilliert. Es verblieben 238 g Rohprodukt (84,3 %ig nach GC).

Gaschromatogramm (HP 5970B, DBWAX 60 N, 60 m, 60 - 240 °C, 4 °C/min.) Das Gemisch bestand aus 2 diastereomeren Enantiomerenpaaren.
**(+/-) 4a** Rₜ = 28,81 Min. = 54,6 %
**(+/-) 4b** Rₜ = 29,24 Min. = 29,7 %
Verhältnis **(+/-) 4a : (+/-) 4b** = 65:35.

Die Bildung der (sensorisch wertvollen) cis-Verbindung **(4a)** war also gegenüber der Bildung der (sensorisch unerwünschten) trans-Verbindung **(4b)** bevorzugt.

Eine Destillation von 100 g Rohprodukt über eine 40 cm Metallfüllkörperkolonne ergab 78 g **(4)** (98 %ig nach GC), Kp₃ₘₘ = 90 - 92 °C.
**(+/-) 4a** Rₜ = 28,8 Min. = 65,7 %
**(+/-) 4b** Rₜ = 29,24 Min. = 32,3 %

Die Destillation von 100 g Rohprodukt an einer Fischer-Spaltrohrkolonne ergab 28,4 g (93 %ig nach GC) **(+/-) 4a** und 18,4 g (89,4 %ig nach GC) **(+/-) 4b**. GC/MS: HP 5970, B, DBWAX 60 N, 60 m, 60 - 240 °C, 4 °C/min.
**(+/-) 4a** Rₜ = 28,85 Min., Kp_{4mbar} = 89 - 90 °C
MS: m/e (%) = 164 (14, M+), 134 (45), 121 (25), 120 (80), 119 (27) 104 (49), 103 (32), 91 (47), 58 (100), 43 (47)
¹³C-NMR (CDCl₃), Varian VXR-300: δ [ppm]: 19.86 (CH₃), 71.93 (CH₂, 78.26, 102.28, (CH), 126.19, 127.91, 128.4 (CH-aromat).
**(+/-) 4b** Rₜ = 29,2 Min., Kp_{4mbar} = 91 - 92 °C
MS: m/e (5) = 164 (15, M+), 134 (38), 121 (33), 120 (70), 104 (52), 103 (41), 91 (46), 77 (24), 58 (100), 43 (46).
¹³C-NMR (CDCl₃), Varian VXR-300: δ [ppm]: 20.35 (CH₃), 72.72 (CH₂), 77.37, 10256 (CH), 125,81, 127.76, 128.48, 128.51 (CH, aromat).

### Beispiel 3

### Herstellung eines Epimerengemischs von (2R,4S)-2-Methyl-4-phenyl-1,3-dioxolan (7) und (2S,4S)-2-Methyl-4-phenyl-1,3-dioxolan (8) - Fig. 2

In einem 100 ml Rührwerk mit Rückflusskühler, Thermometer und Tropftrichter wurde 2 g (14,47 mmol) S(+)-1-Phenyl-1,2-ethandiol (Fa. Fluka) [α D/20° + 39 ± 1 °]), 10 ml Diethylether und 20 mg para-Toluolsulfonsäure vorgelegt, auf 5 - 10 °C heruntergekühlt und bei dieser Temperatur über einen Zeitraum von 15 Min. 0,96 g (21,7 mmol) Acetaldehyd zugetropft. Nachdem 2 Std. bei dieser Temperatur nachgerührt wurde, versetzt man mit 5 ml Kochsalzlösung und arbeitet auf. Das Reaktionsgemisch wurde zweimal mit je 10 ml Ether extrahiert, die vereinigten organischen Phasen mit Sodalösung und Wasser neutralgewaschen, über Na₂SO₄ getrocknet und das Lösungsmittel unter vermindertem Druck abdestilliert. Es verblieben 2,12 g Rohprodukt (96 %ig nach GC).
GC: Bedingungen siehe Beispiel 2
**7** Rₜ = 28,86 Min. = 67,2 %
**8** Rₜ = 29,24 Min. = 28,8 %
Verhältnis **7:8** = 70 : 30

### Beispiel 4

### Herstellung eines Epimerengemischs von (2S,4R)-2-Methyl-4-phenyl-1,3-dioxolan (11) und (2R,4R)-2-Methyl-4-phenyl-1,3-dioxolan (12) - Fig. 3

In einem 100 ml Rührwerk mit Rückflusskühler, Thermometer und Tropoftrichter wurden 2 g (14,47 mmol) R(-)-1-Phenyl-1,2-ethandiol (Fa. Fluka), [α D/20° - 39 ± 1 °]), 10 ml Diethylether, 20 mg para-Toluolsulfonsäure vorgelegt, auf 5 - 10 °C heruntergekühlt und bei dieser Temperatur innerhalb von 15 Min. mit 0,96 g (21,7 mmol) Acetaldehyd versetzt. Anschliessend wurden 2 Std. bei dieser Temperatur nachgerührt, mit 5 ml Kochsalzlösung versetzt und aufgearbeitet. Das Reaktionsgemisch wurde 2 x mit jeweils 10 ml Ether extrahiert, die vereinigten organischen Phasen mit Sodalösung und Wasser neutralgewaschen, über Na₂SO₄ getrocknet und das Lösungsmittel unter vermindertem Druck abdestilliert. Es verblieben 2,09 g Rohprodukt (93,8 %ig nach GC).
GC: Bedingungen siehe Beispiel 2
11 Rₜ = 28,82 Min. = 66,1 %
12 Rₜ = 29,23 Min. = 27,7 %
Verhältnis **11:12** = 70:30

### Beispiel 5

### Alternative Herstellung eines Epimerengemischs von (2R,4S)-2-Methyl-4-phenyl-1,3-dioxolan (7) und (2S,4S)-2-Methyl-4-phenyl-1,3-dioxolan (8) - Fig. 2

In einem 100 ml Rührwerk mit Wasserabscheider, Rückflusskühler und Thermometer wurden 2 g (14,47 mmol) S(+)-1-Phenyl-1,2-ethandiol (Fa. Fluka [α D/20° + 39 ± 1 °]), 20 ml Toluol, 20 mg para-Toluolsulfonsäure und 0,76 g (5,8 mmol) Paraldehyd über einen Zeitraum von 3 Std. unter Rückfluss (110 °C) gerührt. Nach dieser Zeit wurde auf 20 °C heruntergekühlt, mit Sodalösung und Wasser neutralgewaschen. Das Lösungsmittel unter vermindertem Druck abdestilliert. Es verblieben 2,03 g Rohprodukt (91,8 %ig nach GC).
GC: Bedingungen siehe Beispiel 2
**7** Rₜ = 28,81 Min. = 45,9 %
**8** Rₜ = 29,21 Min. = 45,9 %
Verhältnis **7:8** = 50:50

### Beispiel 6

### Alternative Herstellung eines Epimerengemischs aus (2S,4R)-2-Methyl-methyl-4-phenyl-1,3-dioxolan (11) und (2R,4R)-2-Methyl-4-phenyl-1,3-dioxolan (12) - Fig. 3

In einem 100 ml Rührwerk mit Wasserabscheider, Rückflusskühler und Thermometer wurden 2 g (14,47 mmol) R(-)-1-Phenyl-1,2-ethandiol (Fa. Fluka [α D/20° - 39 ± 1 °]), 20 ml Toluol, 20 mg para-Toluolsulfonsäure und 0,76 g (5,8 mmol) Paraldehyd über einen Zeitraum von 3 Std. unter Rückfluss (110 °C) gerührt. Nach dieser Zeit wurde auf 20 °C heruntergekühlt, mit Sodalösung und Wasser neutralgewaschen, das Lösungsmittel unter vermindertem Druck abdestilliert. Es verblieben 2,09 g Rohprodukt (92,6 %ig nach GC).
GC: Bedingungen siehe Beispiel 2
**11** Rₜ = 28,83 Min. = 46,2 %
**12** Rₜ = 29,24 Min. = 46,4 %
Verhältnis **11:12** = 50 : 50

### Beispiel 7

### Isolierung und Analyse der Epimeren aus Beispiel 5 - Fig. 2

2,03 g rohes Acetalgemisch (Epimerengemisch) aus Beispiel 5 (Reinheit nach GC **7**: 45,9 %; **8**: 45,9 %) wurden durch zweifache Flashchromatographie gereinigt.

### Chromatographiebedingungen:

150 g Kieselgel 60, Korngrösse 0,04 - 0,063 mm
(Fa. Merck,Art.-Nr. 9385), Laufmittel Benzin/Essigester 95:5, Einwaage: 2,03 g, Ausbeute: 208 mg Rₜ = 28,8 Min. (7) = 90 %ig nach GC, 156 mg Rₜ = 29,21 Min. (**8**) = 91 %ig nach GC
GC/MS: HP 5970, B, DBWAX 60 N, 60 m, 60 - 240 °C, 4 °C/min.,
**7** Rₜ = 28,4 Min.
MS: m/e (%) = 164 (15, M+), 134 (41), 121 (36), 120 (76), 119 (26), 104 (57), 103 (44), 91 (49), 77 (25), 58 (100), 43 (46),
¹³C-NMR (CDCl₃), Varian VXR-300: δ [ppm]: 19.83 (CH₃), 71.98 (CH₂), 78.37, 102.38 (CH), 126.28. 127.86, 128.48 (CH-aromat).
8 Rt = 29,1 Min.
MS: m/e (%) = 164 (12, M+), 134 (44). 121 (25), 120 (73), 119 (27), 104 (49), 103 (33), 91 (47), 90 (29), 58 (100), 43 (45).
¹³C-NMR (CDCl₃), Varian VXR-300: δ [ppm]: 20.35 (CH₃), 72.77 (CH₂), 77.47, 102.64 (CH), 125,85, 127.89, 128,51, 128.57 (CH-aromat).

### Beispiel 8

### Isolierung und Analyse der Epimeren aus Beispiel 6 - Fig. 3

1 g rohes Acetalgemisch (Epimerengemisch) aus Beispiel 6 wurden durch dreifache Flashchromatographie gereinigt.

### Chromatographiebedingungen:

150 g Kieselgel 60, Korngrösse 0,04 - 0,063 mm, (Fa. Merck, Art.-Nr. 9385). Laufmittel Benzin/Essigester (98:2), Einwaage 1 g;
Ausbeute 78 mg 11.
**11** Rₜ = 28,4 Min.
MS: m/e (%) = 164 (11, M+), 134 (46), 121 (26), 120 (77), 119 (28), 104 (50), 103 (32), 91 (48), 90 (31), 58 (100), 43 (46).
¹³C-NMR (CDCl₃), Varian VXR-300: δ [ppm]: 19.86 (CH₃), 71.98 (CH₂), 78.34, 102.36 (CH), 126.23, 127.99, 128.48 (CH-aromat).
Ausbeute: 65 mg 12
**12** Rₜ = 29,0 Min.
MS: m/e (5) = 164 (12, M+), 134 (41), 121 (35), 120 (73), 119 (24), 104 (54), 103 (42), 91 (46), 77 (24), 58 (100), 43 (44).
¹³C-NMR (CDCl₃), Varian VXR-300: δ [ppm]: 20.34 (CH₃), 72.78 CH₂), 77.49, 102.66 (CH), 125.87, 127.92, 128.53, 128.56 (CH-aromat).

### Beispiel 9

### Alternative Herstellung von (±)-2-Methyl-4-phenyl-1,3-dioxolan (4) (Reaktionstemperatur: 110 °C) - Fig. 1

In einem 1 l Rührwerk mit Wasserabscheider, Rückflusskühler und Thermometer wurden 100 g (0,63 mol) (±)-1-Phenyl-1,2-ethandiol (3) (87 %ig nach GC) nach Beispiel 1, 200 ml Toluol, 32 g (0,24 mol) Paraldehyd und 1 g para-Toluolsulfonsäure vorgelegt, zum Sieden (110 °C) erhitzt und über einen Zeitraum von 4 Std. unter Rückfluss gerührt. Es wurden insgesamt 10 ml Wasser abgespalten. Nach dieser Zeit wurde auf 20 °C heruntergekühlt, mit Sodalösung und Wasser neutralgewaschen, das Lösungsmittel unter vermindertem Druck abdestilliert. Es verblieben 103 g Rohprodukt (85,3 %ig nach GC). GC-Bedingungen siehe Beispiel 2.
**(+/-) 4a** Rₜ = 28,8 Min. = 43,5 %
**(+/-) 4b** Rₜ = 29,2 Min. = 41,8 %
Verhältnis **(+/-) 4a : (+/-) 4b** = 51:49.

Es wurden also nahezu gleiche Mengen der (sensorisch wertvollen) cis- und der (sensorisch unerwünschten) trans-Verbindung erzeugt.

### Beispiel 10

### Alternative Herstellung von (±)-2-Methyl-4-phenyl-1,3-dioxolan (4) (Reaktionstemperatur: 160 °C) - Fig. 1

In einem 500 ml Rührwerk mit Wasserabscheider, Rückflusskühler und Thermometer wurden 69 g (0,45 mol) (±)-1-Phenyl-1,2-ethandiol (**3**) (90 %ig nach GC; hergestellt gemäß Beispiel 1, 150 ml Xylol, 31 g (0,23 mol) Paraldehyd und 0,6 g para-Toluolsulfonsäure vorgelegt, zum Sieden (160 °C) erhitzt und über einen Zeitraum von 4 Std. unter Rückfluss gerührt. Es wurden insgesamt 7,2 ml Wasser abgespalten. Nach dieser Zeit wurde auf 20 °C heruntergekühlt, mit Sodalösung und Wasser neutralgewaschen, das Lösungsmittel unter vermindertem Druck abdestilliert. Es verblieben 72 g Rohprodukt (87,8 %ig nach GC).
GC-Bedingungen: siehe Beispiel 2
**(+/-) 4a** Rₜ = 28,81 Min. = 33,12 %
**(+/-) 4b** Rₜ = 29,32 Min. = 54,68 %
Verhältnis **(+/-)4a : (+/-) 4b** = 38 : 62

Es wurden also bei der gewählten hohen Reaktionstemperatur deutlich geringere Mengen an der sensorisch wertvollen cis-Verbindung erzeugt.

### Beispiel 11

### Alternative Herstellung von (±)-2-Methyl-4-phenyl-1,3-dioxolan (4) (Reaktionstemperatur: -20°C bis -15°C) - Fig. 1

In einem 100 ml Rührwerk mit Rückflusskühler, Thermometer und Tropftrichter wurden 2,76 g (20 mmol) (±)-1-Phenyl-1,2-ethandiol **(3)** (90 %ig nach GC), hergestellt nach Beispiel 1, 10 ml Ether, 20 mg para-Toluolsulfonsäure vorgelegt, auf - 20 °C heruntergekühlt und innerhalb von 15 Min. 1,32 g (30 mmol) Acetaldehyd zugetropft, wobei eine Temperatur von - 15 °C im Reaktionsgemisch nicht überschritten wurde. Anschliessend wurde über einen Zeitraum von 6 Std. bei - 20 °C bis - 15 °C nachgerührt, mit Sodalösung und Wasser neutralgewaschen, über Natriumsulfat getrocknet und das Lösungsmittel unter vermindertem Druck abdestilliert. Es verblieben 2,91 g Rohprodukt (89,2 %ig nach GC).
GC-Bedingungen: siehe Beispiel 2
**(+/-) 4a** Rₜ = 28,80 Min. = 71,4 %
**(+/-) 4b** Rₜ = 29,20 Min. = 17,8 %
Verhältnis **(+/-) 4a : (+/-) 4b** = 80 : 20

Die Bildung der sensorisch wertvollen cis-Verbindung war also bei der gewählten niedrigen Reaktionstemperatur stark bevorzugt.

### Beispiel 12

### Alternative Herstellung von (±)-2-Methyl-4-phenyl-1,3-dioxolan (4) (Reaktionstemperatur: -70°C) - Fig. 1

In einem 100 ml Rührwerk mit Rückflusskühler, Thermometer und Tropftrichter wurden 2,76 g (20 mmol) (±)-1-Phenyl-1,2-ethandiol (3) (90 %ig nach GC), hergestellt nach Beispiel 1, 10 ml Ether, 20 mg para-Toluolsulfonsäure vorgelegt, auf - 70 °C heruntergekühlt und bei dieser Temperatur innerhalb von 30 Minuten 1,32 g (30 mmol) Acetaldehyd zugetropft. Anschliessend wurde über einen Zeitraum von 30 Std. bei - 70 °C nachgerührt, mit Sodalösung und Wasser neutralgewaschen, über Na₂SO₄ getrocknet und das Lösungsmittel unter vermindertem Druck abdestilliert. Es verblieben 2,83 g Rohprodukt (78,3 %ig nach GC).
GG-Bedingungen: siehe Beispiel 2
**(+/-) 4a** Rₜ = 28,78 Min. = 70,5 %
**(+/-) 4b** Rₜ = 29,18 Min. = 7,8 %
Verhältnis **(+/-) 4a : (+/-) 4b** = 90:10

Die Bildung der sensorisch wertvollen cis-Verbindung war also bei der gewählten sehr niedrigen Reaktionstemperatur überaus stark bevorzugt.

### Beispiel 13

### Geruchsbeschreibung der Acetale 4, (+/-) 4a, (+/-) 4b, 7, 8, 11, 12

Die Geruchsbewertung wurde von einem Expertengremium anhand 10 %iger ethanolischer Lösungen der Acetale unter Verwendung von Riechstreifen vorgenommen.
Destillat - Metallfüllkörperkolonne **4** aus Beispiel 2
   stark, blumig, Hyacinthe, Styrol, Indol, grün, narkotisch, Schokoladen-Note Nachgeruch: holzig, ozonig
Destillat-Fischer-Spaltrohrkolonne **(+/-) 4a** aus Beispiel 2
   stark, Indol, animalisch, Styrol, Styrax, Hyazinthe
Destillat -Fischer-Spaltrohrkolonne **(+/-) 4b** aus Beispiel 2
   schwach, animalisch, ausgeprägte Schokoladen-Note
**7** aus Beispiel 7:
   stark, klar, Indol, Rose-Jasmin, am stärksten und am besten riechendes Enantiomer
**8** aus Beispiel 7:
   blumig, holzig, Diphenylether, Rose, animalisch, Schokoladen-Note
**11** aus Beispiel 8:
   Styrol, Indol, Skatol, Hyazinthe
**12** aus Beispiel 8:
   Blumig, Flieder, holzig, Oranger-liquid, Schokoladen-Note

### Beispiel 14

### Geruchsschwellenbestimmung der Acetale 7, 8, 11, 12

Die Geruchsschwellenbestimmung wurde von einem Expertengremium von 20 Testpersonen vorgenommen. Es wurde die Geruchsschwelle in Wasser bestimmt.
**7** aus Beispiel 7
   Mittelwert: 125 µg/L
**8** aus Beispiel 7
   Mittelwert: 384 µg/L
**11** aus Beispiel 8
   Mittelwert: 384 µg/L
**12** aus Beispiel 8
   Mittelwert: 589 µg/L

## Patentansprüche

1. (2R,4S)-2-Methyl-4-phenyl-1,3-dioxolan oder
(2S,4R)-2-Methyl-4-phenyl-1,3-dioxolan,
insbesondere zur Verwendung als sensorisch aktive Substanz.

2. Verwendung einer Substanz aus der Gruppe umfassend (2S,4R)-2-Methyl-4-phenyl-1,3-dioxolan,
(2R,4S)-2-Methyl-4-phenyl-1,3-dioxolan und
deren Mischungen
als sensorisch aktive Substanz.

3. Gemisch, umfassend einen Gewichtsteil an
(2S,4R)-2-Methyl-4-phenyl-1,3-dioxolan und/oder
(2R,4S)-2-Methyl-4-phenyl-1,3-dioxolan
und weniger als zwei Gewichtsteile an
(2R,4R)-2-Methyl-4-phenyl-1,3-dioxolan und/oder
(2S,4S)-2-Methyl-4-phenyl-1,3-dioxolan,
insbesondere zur Verwendung als sensorisch aktive Substanz.

4. Gemisch, umfassend einen Gewichtsteil an
(2S,4R)-2-Methyl-4-phenyl-1,3-dioxolan und/oder
(2R,4S)-2-Methyl-4-phenyl-1,3-dioxolan
und weniger als einen Gewichtsteil an
(2R,4R)-2-Methyl-4-phenyl-1,3-dioxolan und/oder
(2S,4S)-2-Methyl-4-phenyl-1,3-dioxolan,
insbesondere zur Verwendung als sensorisch aktive Substanz.

5. Verfahren zur Herstellung eines Gemisches nach einem der Ansprüche 3 oder 4,
**dadurch gekennzeichnet, daß** Acetaldehyd bei einer Temperatur von 20°C oder weniger mit racemischem oder enantiomerenreinem 1-Phenyl-1,2-ethandiol acetalisiert wird.

6. Verfahren zur Herstellung von
(2S,4R)-2-Methyl-4-phenyl-1,3-dioxolan und/oder
(2R,4S)-2-Methyl-4-phenyl-1,3-dioxolan
**dadurch gekennzeichnet, daß**
(2S,4S)-2-Methyl-4-phenyl-1,3-dioxolan und/oder
(2R,4R)-2-Methyl-4-phenyl-1,3-dioxolan,
gelöst in einem Lösungsmittel, auf eine Temperatur von 20°C oder weniger, vorzugsweise 0°C oder weniger, gebracht werden bzw. wird.

7. Verwendung eines Gemisches nach einem der Ansprüche 3 oder 4 als Duftstoff.

## Claims

1. (2R,4S)-2-Methyl-4-phenyl-1,3-dioxolane or
(2S,4R)-2-methyl-4-phenyl-1,3-dioxolane,
in particular for use as substance having a sensory action.

2. Use of a substance from the group comprising
(2S,4R)-2-methyl-4-phenyl-1,3-dioxolane,
(2R,4S)-2-methyl-4-phenyl-1,3-dioxolane and
mixtures thereof
as substance having a sensory action.

3. Mixture comprising one part by weight of
(2S,4R)-2-methyl-4-phenyl-1,3-dioxolane and/or
(2R,4S)-2-methyl-4-phenyl-1,3-dioxolane
and less than two parts by weight of
(2R,4R)-2-methyl-4-phenyl-1,3-dioxolane and/or
(2S ,4S)-2-methyl-4-phenyl-1,3-dioxolane,
in particular for use as substance having a sensory action.

4. Mixture comprising one part by weight of
(2S,4R)-2-methyl-4-phenyl-1,3-dioxolane and/or
(2R,4S)-2-methyl-4-phenyl-1,3-dioxolane
and less than one part by weight of
(2R,4R)-2-methyl-4-phenyl-1,3-dioxolane and/or
(2S,4S)-2-methyl-4-phenyl-1,3-dioxolane,
in particular for use as substance having a sensory action.

5. Method for the preparation of a mixture according to one of Claims 3 or 4,
**characterised in that** acetaldehydeis acetalated with racemic or enantiomerically pure 1-phenyl-1,2-ethanediol at a temperature of 20°C or below.

6. Method for the preparation of
(2S,4R)-2-methyl-4-phenyl-1,3-dioxolane and/or
(2R,4S)-2-methyl-4-phenyl-1,3-dioxolane
**characterised in that**
(2S,4S)-2-methyl-4-phenyl-1,3-dioxolane and/or
(2R,4R)-2-methyl-4-phenyl-1,3-dioxolane,
dissolved in a solvent, are or is brought to a temperature of 20°C or below, preferably 0°C or below.

7. Use of a mixture according to one of Claims 3 or 4 as a fragrance.

## Revendications

1. (2R,4S)-2-méthyl-4-phényl-1,3-dioxolane ou (2S,4R)-2-méthyl-4-phényl-1,3-dioxolane,
en particulier pour utilisation en tant que substance sensorielle active.

2. Utilisation d'une substance choisie dans le groupe comprenant :
le (2S,4R)-2-méthyl-4-phényl-1,3-dioxolane,
le (2R,4S)-2-méthyl-4-phényl-l,3-dioxolane et
le mélange de ceux-ci
en tant que substance sensorielle active.

3. Mélange comprenant une part en poids de
(2S,4R)-2-méthyl-4-phényl-l,3-dioxolane et/ou
(2R,4S)-2-méthyl-4-phényl-1,3-dioxolane
et moins que deux parts en poids de
(2R,4R)-2-méthyl-4-phényl-1,3-dioxolane et/ou
(2S,4S)-2-méthyl-4-phényl-1,3-dioxolane
en particulier pour utilisation en tant que substance sensorielle active.

4. Mélange comprenant une part en poids de
(2S,4R)-2-méthyl-4-phényl-1,3-dioxolane et/ou
(2R,4S)-2-méthyl-4-phényl-1,3-dioxolane
et moins qu'une part en poids de
(2R,4R)-2-méthyl-4-phényl-1,3-dioxolane et/ou
(2S,4S)-2-méthyl-4-phényl-1,3-dioxolane
en particulier pour utilisation en tant que substance sensorielle active.

5. Procédé pour la fabrication d'un mélange selon l'une quelconque des revendications 3 ou 4,
**caractérisé en ce que** l'acétaldéhyde est soumis à une acétalisation avec du 1-phényl-1,2-éthandiol racémique ou énantiopur à une température de 20 °C ou moins.

6. Procédé pour la fabrication de
(2S,4R)-2-méthyl-4-phényl-1,3-dioxolane et/ou
(2R,4S)-2-méthyl-4-phényl-1,3-dioxolane
**caractérisé en ce que**
(2S,4S)-2-méthyl-4-phényl-1,3-dioxolane et/ou
(2R,4R)-2-méthyl-4-phényl-1,3-dioxolane
dissous dans un solvant, sont portés, respectivement est porté, à une température de 20 °C ou moins, de préférence 0 °C ou moins.

7. Utilisation d'un mélange selon l'une quelconque des revendications 3 ou 4 en tant que parfum.
